# EUROPÄISCHE PATENTANMELDUNG

(11) **EP 2 028 226 A1**
(43) Veröffentlichungstag der Anmeldung: **25.02.2009**
(21) Anmeldenummer: 08014581.6
(22) Anmeldetag: 16.08.2008
(51) Int. Cl.: C08K 5/3435, C07D 295/02, C09K 21/12

(54) **Piperazinpolyphosphat, Verfahren zu seiner Herstellung und seine Verwendung**

(30) Priorität: 22.08.2007 DE 102007039559
(71) Anmelder: Clariant International Ltd., 4132 Muttenz (CH)
(72) Erfinder: Eisenträger, Frank, Dr., 08812 St Pere de Ribes (ES); Hörold, Sebastian, Dr., 86420 Diedorf (DE); Wanzke, Wolfgang, Dr., 86161 Augsburg (DE); Hill, Michael, Dr., 50827 Köln (DE)
(74) Vertreter: Paczkowski, Marcus

(57) **Zusammenfassung**

Die Erfindung betrifft ein Piperazinpolyphosphat, dadurch gekennzeichnet, dass es eine mittlere Kettenlänge von 2,2 bis 10 Phosphateinheiten aufweist, ein Verfahren zu seiner Herstellung und seine Verwendung.

## Beschreibung

Die Erfindung betrifft Piperazinpolyphosphat, Verfahren zu seiner Herstellung und seine Verwendung.

In der WO-2005/037 806 wird ein Piperazinpyrophosphat beschrieben, das durch Kondensation zweier Piperazinphosphat-Einheiten erhalten wird. Gleichzeitig wird beschrieben, dass Verunreinigungen (mit Piperazinphosphat) zu einer Erniedrigung der Zersetzungstemperatur dieses Produktes führen. Die reinsten Piperazinpyrophosphate haben eine Zersetzungstemperatur von 324 °C (5 % Gewichtsverlust, GV).

Die DE-A-101 45 093 beschreibt, dass ein Piperazinpolyphosphat aus Phosphorpentoxid und Piperazin (im molaren Verhältnis 1:2) und einem Wasserspender hergestellt werden kann.

Die WO-2006/027 340 beschreibt, dass bei Polyphosphatsalzen mit Aminbasen eine niedrige Wasserlöslichkeit und eine niedrige Leitfähigkeit für die Anwendung als Flammschutzmittel von Bedeutung sind.

Die EP-A-1 277 794 beschreibt die Flammschutzwirkung eines Melaminpyrophosphats mit einem Umsetzungsprodukt eines Gemisches von Phosphorsäure (50 %), Pyrophosphorsäure (30 %), Triphosphorsäure (15 %) und andere Polyphosphorsäuren (5 %) mit Piperazin im molaren Verhältnis 1:1.

Bei medizinischen Anwendungen, z. B. als Entwurmungsmittel steht die Wasserlöslichkeit in direktem Zusammenhang mit der Verfügbarkeit im Körper. Mit einer niedrigen Wasserlöslichkeit können lang anhaltende Wirkungen erzielt werden. In der Anwendung von Piperazinpolyphosphat als Flammschutzmittel limitiert die Zersetzungstemperatur das Verarbeitungsfenster bei der Einbringung in die Polymermasse. Eine hohe Zersetzungstemperatur dagegen ermöglicht den Einsatz in einem breiten Spektrum von Polymeren unter verschiedensten Bedingungen.

Die bisher beschriebenen Piperazinphosphat-Verbindungen und Piperazinpolyphosphate weisen aber bezüglich (i. Allg. zu hoher) Wasserlöslichkeit, Säurezahl, Leitfähigkeit und (i. Allg. zu niedriger) Zersetzungstemperatur Nachteile auf, die einen breiteren Einsatz verhindern.

Es bestand somit die Aufgabe, ein Piperazinpolyphosphat mit niedriger Wasserlöslichkeit, niedriger Leitfähigkeit, niedriger Säurezahl und hoher Zersetzungstemperatur zu synthetisieren.

Bezüglich des Verfahrens bestand die Aufgabe, dies auf Basis von günstigen Rohstoffen und in einem einzigen Reaktionsschritt durchzuführen, ohne dass vielfältige Nebenprodukte entstehen.

Überraschend wurde nun gefunden, dass Piperazinpolyphosphate mit einer Kettenlänge von 2,2 bis 10 bei einem molaren Piperazin zu Phosphorpentoxidverhältnis von 0,9 bis 1,5 zu 1 eine besonders niedrige Wasserlöslichkeit, Säurezahl und Leitfähigkeit, günstige Farbzahlen, niedrige Restfeuchten und eine hohe Zersetzungstemperatur aufweisen.

Die Erfindung betrifft daher ein Piperazinpolyphosphat, das eine mittlere Kettenlänge von 2,2 bis 10 Phosphateinheiten, eine Kettenlängenverteilung mit:

| | |
|---|---|
| Kettenlänge 1 | 0,5 bis 10 % |
| Kettenlänge 2 | 2 bis 30 % |
| Kettenlänge 3 | 4 bis 30 % |
| Kettenlänge 4 | 0 bis 90 % |
| Kettenlänge 5 | 1 bis 25 % |
| Kettenlänge 6 | 1 bis 25 % |
| Kettenlänge 7 | 1 bis 40 % |
| Kettenlänge 8 und darüber | 0,5 bis 40 %, |

die Säurezahl des Filtrats einer 10%igen Suspension davon zwischen 200 und 400 mg KOH/g liegt und das eine Wasserlöslichkeit von maximal 7 % aufweist.

Besonders bevorzugt weist das Piperazinpolyphosphat folgende Kettenlängenverteilung auf:

| | |
|---|---|
| Kettenlänge 1 | 0,5 bis 5 % |
| Kettenlänge 2 | 2 bis 20% |
| Kettenlänge 3 | 4 bis 20 % |
| Kettenlänge 4 | 30 bis 90 % |
| Kettenlänge 5 | 1 bis 15 % |
| Kettenlänge 6 | 1 bis 15 % |
| Kettenlänge 7 | 1 bis 30 % |
| Kettenlänge 8 und darüber | 0,5 bis 30 %. |

Die Summe der Kettenlängenverteilungen betragen daher immer 100 %.

Bevorzugt weist eine 10 %ige Suspension des Piperazinpolyphosphats in Wasser einen pH-Wert zwischen 2,0 und 5,5 auf.

Die Säurezahl des Filtrats einer 10 %igen Suspension des Piperazinpolyphosphats liegt bevorzugt zwischen 200 und 400 mg KOH/g.

Die Erfindung betrifft auch ein Verfahren zu Herstellung von Piperazinpolyphosphat, dadurch gekennzeichnet, dass flüssiges und/oder gasförmiges Piperazin und Polyphosphorsäure miteinander umgesetzt werden.

In einer bevorzugten Ausführungsform ist das Verfahren zur Herstellung von Piperazinpolyphosphat dadurch gekennzeichnet, dass Polyphosphorsäure und Piperazin in einem Lösungsmittel miteinander umgesetzt werden.

In einer anderen Ausführungsform ist das Verfahren zur Herstellung von Piperazinpolyphosphat dadurch gekennzeichnet, dass ein Salz einer Aminbase der Polyphosphorsäure in wässriger Lösung mit Piperazin umgesetzt wird.

Die Erfindung betrifft auch die Verwendung von Piperazinpolyphosphat nach mindestens einem oder mehreren der Ansprüche 1 bis 4 als Flammschutzmittel, insbesondere für Klarlacke und Intumeszenzbeschichtungen, Flammschutzmittel für Holz und andere cellulosehaltige Produkte, für oder in Kunststoffen, Holz, Papier und Textil sowie in Beschichtungen für Stahl- und Holzbrandschutz; als reaktives und/oder nicht reaktives Flammschutzmittel für Polymere, zur Herstellung von flammgeschützten Polymerformmassen, zur Herstellung von flammgeschützten Polymerformkörpern, -Filmen, -Fäden und -Fasern und/oder zum flammhemmend Ausrüsten von Polyester und Cellulose-Rein- und Mischgeweben durch Imprägnierung.

Schließlich betrifft die Erfindung auch die Verwendung von Piperazinpolyphosphat nach mindestens einem oder mehreren der Ansprüche 1 bis 4
- als Zwischenprodukt für weitere Synthesen,
- als Binder,
- als Vernetzer bzw. Beschleuniger beim Aushärten von Epoxyharzen, Polyurethanen, ungesättigten Polyesterharzen,
- als Polymerstabilisatoren,
- als Pflanzenschutzmittel,
- als Therapeutikum oder Additiv in Therapeutika für Menschen und Tiere,
- als Sequestrierungsmittel,
- als Mineralöl-Additiv,
- als Korrosionsschutzmittel,
- in Wasch- und Reinigungsmittelanwendungen,
- in Elektronikanwendungen

Die Erfindung betrifft auch eine flammgeschützte thermoplastische und/oder duroplastische Polymerformmasse, enthaltend 0,5 bis 45 Gew.-% Piperazinpolyphosphat nach mindestens einem oder mehreren der Ansprüche 1 bis 4, 0,5 bis 95 Gew.-% thermoplastisches Polymer oder Mischungen derselben, 0 bis 55 Gew.-% Additive und 0 bis 55 Gew.-% Füllstoff bzw. Verstärkungsmaterialien, wobei die Summe der Komponenten 100 Gew.-% beträgt.

Die Erfindung betrifft zuletzt auch flammgeschützte thermoplastische und/oder duroplastische Polymer-Formkörper, -Filme, -Fäden und Fasern, enthaltend 0,5 bis 45 Gew.-% Piperazinpolyphosphat nach mindestens einem oder mehreren der Ansprüche 1 bis 4, 0,5 bis 95 Gew.-% thermoplastisches Polymer oder Mischungen derselben, 0 bis 55 Gew.-% Additive und 0 bis 55 Gew.-% Füllstoff bzw. Verstärkungsmaterialien, wobei die Summe der Komponenten 100 Gew.-% beträgt.

Beim erfindungsgemäßen Piperazinpolyphosphat kann der pH-Wert einer 10 %igen Suspension davon in Wasser auch zwischen 3 und 6 liegen.

Beim erfindungsgemäßen Piperazinpolyphosphat kann die Säurezahl des Filtrats einer 10 %igen Suspension davon auch zwischen 100 mg KOH/g und 500 mg KOH/g liegen.

Das erfindungsgemäße Piperazinpolyphosphat zeigt eine Wasserlöslichkeit kleiner als 5 % und besonderes bevorzugt kleiner als 4 %.

Das erfindungsgemäße Piperazinpolyphosphat weist eine Leitfähigkeit von 1000 bis 12000 µS/cm in einer 10 %igen Suspension auf.

Beim erfindungsgemäßen Piperazinpolyphosphat liegt die Temperatur bei 5 % Gewichtsverlust - als Maß für die Thermostabilität - zwischen 290 °C und 400 °C, bevorzugt zwischen 300 °C und 380 °C und besonders bevorzugt zwischen 320 °C und 370 °C.

Bevorzugt weist das erfindungsgemäße Piperazinpolyphosphat einen mittleren Teilchendurchmesser (d50) von kleiner 100 µm, bevorzugt kleiner 50 µm und besonders bevorzugt < 30 µm auf.

Bevorzugt beträgt beim erfindungsgemäßen Piperazinpolyphosphat die Restfeuchte kleiner 1 %, bevorzugt kleiner 0,5 % und besonders bevorzugt kleiner 0,1 %.

Ferner betrifft die Erfindung auch ein Piperazinpolyphosphat, dadurch gekennzeichnet, dass ein Piperazin-Phosphorpentoxid-Verhältnis (gleichbedeutend mit dem N:P Verhältnis) von 0,9-1,5 zu 1 vorliegt. Bevorzugt ist ein Piperazinpolyphosphat mit einem N:P Verhältnis von 0,9-1,2 zu 1, besonders bevorzugt ist ein N:P Verhältnis von 0,9-1,0 zu 1.

Ferner betrifft die Erfindung ein Piperazinpolyphosphat, dadurch gekennzeichnet, dass die Farbzahlen des Produkts sich in folgenden Grenzen bewegen: Die Farbzahl L liegt zwischen 70 und 100, besonders bevorzugt zwischen 80 und 100. Die Farbzahl a liegt zwischen 0 und 3,0, besonders bevorzugt zwischen 0 und 1,5. Die Farbzahl b liegt zwischen 0 und 8, bevorzugt zwischen 0 und 6 und besonders bevorzugt zwischen 0 und 5. Gegenstand der vorliegenden Erfindung ist ferner ein Verfahren zur Herstellung von Piperazinpolyphosphat. Das erfindungsgemäße Verfahren wird vorzugsweise in einem Sprühturm ausgeführt, in dem flüssiges und/oder gasförmiges Piperazin und Polyphosphorsäure miteinander zur Reaktion gebracht werden. Die Temperatur der Polyphosphorsäure beträgt dabei 50 bis 250 °C, besonders bevorzugt 100 °C bis 200 °C, die Temperatur des Piperazin beträgt 110°C bis 150°C.

Ferner kann man erfindungsgemäße Verfahren derart ausführen, dass Polyphosphorsäure in einem geeigneten Aggregat mit gasförmigen Piperazin zur Reaktion gebracht wird.

Ferner kann man erfindungsgemäße Verfahren derart ausführen, dass Polyphosphorsäure und Piperazin in einem Lösungsmittel umgesetzt werden. Dabei werden vorzugsweise Lösungsmittel benutzt, die Piperazin lösen.

Ebenso kann ein inertes Lösungsmittel bei Temperaturen nahe oder über dem Schmelzpunkt von Piperazin benutzt werden. Das Produkt wird anschließend durch Filtration oder Destillation vom Lösungsmittel abgetrennt.

Ferner kann man das erfindungsgemäße Verfahren derart ausführen, dass in einem Reaktor mit Misch- und Mahlorganen Polyphosphorsäure und Piperazin flüssig und/oder gasförmig gleichzeitig zugegeben werden. Dabei kann der Reaktor leer sein oder bereits eine Fertigproduktschüttung von Piperazinpolyphosphat beinhalten.

Das erfindungsgemäße Verfahren kann sowohl kontinuierlich als auch diskontinuierlich ausgeführt werden.

Ferner kann man das erfindungsgemäße Verfahren derart ausführen, dass ein Salz einer Aminbase der Polyphosphorsäure (Ammoniumsalze der Polyphosphorsäure, z. B. Ammoniumpolyphosphat) in wässriger Lösung mit Piperazin umgesetzt wird. Das Produkt wird abfiltriert und getrocknet.

Bevorzugt wird die Umsetzung von Piperazin und Polyphosphorsäure unter dem eigenen Dampfdruck des Piperazins und/oder des Lösungsmittels durchgeführt.

Besonders erfolgt die Umsetzung in einer Atmosphäre, die weitere gasförmige Bestandteile wie zum Beispiel Stickstoff, Sauerstoff, Argon etc. enthält.

Bevorzugt erfolgt die Umsetzung bei einem Partialdruck des Piperazins von 0.01 bis 100 bar.

Besonders bevorzugt erfolgt die Umsetzung bei einem Partialdruck des Piperazins von 0.1 bis 10 bar.

Bevorzugt wird die Umsetzung bei einer Temperatur von -20 bis 340 °C durchgeführt.

Besonders bevorzugt erfolgt die Umsetzung bei einer Temperatur von 20 bis 270 °C.

Bevorzugt erfolgt die Umsetzung bei einem Gesamtdruck von 1 bis 100 bar. Bevorzugt erfolgt die Umsetzung in einem Piperazinpolyphosphorsäure-Verhältnis, gekennzeichnet durch ein Piperazinphosphorpentoxid-Verhältnis (gleichbedeutend mit dem N:P Verhältnis) von 0,9-1,5 zu 1. Besonders bevorzugt ist ein N:P Verhältnis von 0,9-1,2 zu 1. Ganz besonders bevorzugt ist ein N:P Verhältnis von 0,9-1,0 zu 1.

Geeignete Lösungsmittel sind Wasser, Alkohole, wie z. B. Methanol, Ethanol, i-Propanol, n-Propanol, n-Butanol, i-Butanol, t-Butanol, n-Amylalkohol, i-Amylalkohol, t-Amylalkohol, n-Hexanol, n-Octanol, i-Octanol, n-Tridecanol, Benzylalkohol etc. Bevorzugt sind weiterhin Glycole wie z.B. Ethylenglycol, 1,2-Propandiol, 1,3-Propandiol, 1,3-Butandiol, 1,4-Butandiol, Diethylenglycol etc.; aliphatische Kohlenwasserstoffe wie Pentan, Hexan, Heptan, Octan, und Petrolether, Petroleumbenzin, Kerosin, Petroleum, Paraffinöl etc.; aromatische Kohlenwasserstoffe wie Benzol, Toluol, Xylol, Mesitylen, Ethylbenzol, Diethylbenzol etc.; Halogenkohlenwasserstoffe wie Methylenchlorid, Chloroform, 1,2-Dichloroethan, Chlorobenzol, Tetrachlorkohlenstoff, Tetrabromoethylen etc.; alicyclische Kohlenwasserstoffe wie Cyclopentan, Cyclohexan, und Methylcyclohexan etc.; Ether wie Anisol (Methylphenylether), t-Butylmethylether, Dibenzylether, Diethylether, Dioxan, Diphenylether, Methylvinylether, Tetrahydrofuran, Triisopropylether etc.; Glycolether wie Diethylenglycoldiethylether, Diethylenglycoldimethylether (Diglyme), Diethylenglycolmonobutylether, Diethylenglycolmonomethylether, 1,2-Dimethoxyethan (DME Monoglyme), Ethylenglycolmonobutylether, Triethylenglycoldimethylether (Triglyme), Triethylenglycolmonomethylether etc.; Ketone wie Aceton, Diisobutylketon, Methyl-n-propylketon; Methylethylketon, Methyl-i-butylketon etc; Ester wie Methylformat, Methylacetat, Ethylacetat, n-Propylacetat und n-Butylacetat etc.; Carbonsäuren wie Ameisensäure, Essigsäure, Propionsäure, Buttersäure etc. Eine oder mehrere dieser Verbindungen können allein oder in Kombination eingesetzt werden.

Geeignete Lösungsmittel umfassen zudem Piperazin und Polyphosphorsäure. Diese bieten Vorteile in Form einer höheren Raum-Zeit-Ausbeute.

Bei allen beschriebenen Verfahren ist es günstig, das erhaltene Produkt zu tempern. Bevorzugt wird dies bei Temperaturen zwischen 150 °C und 290 °C durchgeführt, besonders bevorzugt bei Temperaturen zwischen 170 °C und 250 °C.

Durch geeignete Wahl der eingesetzten Polyphosphorsäure (Konzentrationsgrad) und der Reaktionstemperatur erhält man spezifische Kettenlängenverteilungen. Hierbei nimmt der Anteil an längerkettigem Produkt mit steigender Konzentration der Polyphosphorsäure zu. Ein Einfluss der Temperatur auf die Kettenlängenverteilung ist ebenfalls gegeben.

Mit 76 %iger Polyphosphorsäure besitzt die Hauptkomponente des Piperazinpolyphosphats eine Kettenlänge von 1, mit 81 %iger Polyphosphorsäure von 2 und ab 84 %iger Polyphosphorsäure von 4, wobei mit 85- und 86 %iger Polyphosphorsäure der Anteil an Komponenten mit Kettenlängen, die größer sind als 4 sind, zunimmt. Dies ist wie folgt zu erkennen:

Bevorzugt weist das Piperazinpolyphosphat hergestellt aus 76 %iger Polyphosphorsäure und Piperazin folgende Kettenlängenverteilung auf:

| | |
|---|---|
| Kettenlänge 1 | 55 bis 85 % |
| Kettenlänge 2 | 15 bis 45 % |
| Kettenlänge 3 | 0 bis 5 % |
| Kettenlänge 4 | 0 bis 5 % |
| Kettenlänge 5 | 0 bis 2 % |
| Kettenlänge 6 | 0 bis 2 % |
| Kettenlänge 7 | 0 bis 1 % |
| Kettenlänge 8 und darüber | 0 bis 1 %. |

Bevorzugt weist das Piperazinpolyphosphat hergestellt aus 81 %iger Polyphosphorsäure und Piperazin folgende Kettenlängenverteilung auf:

| | |
|---|---|
| Kettenlänge 1 | 0 bis 10 % |
| Kettenlänge 2 | 70 bis 100 % |
| Kettenlänge 3 | 0 bis 10 % |
| Kettenlänge 4 | 0 bis 20 % |
| Kettenlänge 5 | 0 bis 5 % |
| Kettenlänge 6 | 0 bis 5 % |
| Kettenlänge 7 | 0 bis 2 % |
| Kettenlänge 8 und darüber | 0 bis 2 %. |

Bevorzugt weist das Piperazinpolyphosphat hergestellt aus 84 %iger Polyphosphorsäure und Piperazin folgende Kettenlängenverteilung auf:

| | |
|---|---|
| Kettenlänge 1 | 0 bis 10 % |
| Kettenlänge 2 | 20 bis 60 % |
| Kettenlänge 3 | 0 bis 15 % |
| Kettenlänge 4 | 20 bis 60 % |
| Kettenlänge 5 | 0 bis 10 % |
| Kettenlänge 6 | 0 bis 10 % |
| Kettenlänge 7 | 0 bis 10 % |
| Kettenlänge 8 und darüber | 0 bis 5 %. |

Bevorzugt weist das Piperazinpolyphosphat hergestellt aus 85 %iger Polyphosphorsäure und Piperazin folgende Kettenlängenverteilung auf:

| | |
|---|---|
| Kettenlänge 1 | 0 bis 10 % |
| Kettenlänge 2 | 0 bis 25 % |
| Kettenlänge 3 | 0 bis 20 % |
| Kettenlänge 4 | 50 bis 90 % |
| Kettenlänge 5 | 0 bis 10 % |
| Kettenlänge 6 | 0 bis 10 % |
| Kettenlänge 7 | 0 bis 10 % |
| Kettenlänge 8 und darüber | 0 bis 5 %. |

Bevorzugt weist das Piperazinpolyphosphat hergestellt aus 86 %iger Polyphosphorsäure und Piperazin folgende Kettenlängenverteilung auf:

| | |
|---|---|
| Kettenlänge 1 | 0 bis 5 % |
| Kettenlänge 2 | 0 bis 15 % |
| Kettenlänge 3 | 0 bis 20 % |
| Kettenlänge 4 | 55 bis 75 % |
| Kettenlänge 5 | 0 bis 15 % |
| Kettenlänge 6 | 0 bis 15 % |
| Kettenlänge 7 | 0 bis 30 % |
| Kettenlänge 8 und darüber | 0 bis 30 %. |

Die Summe der Kettenlängenverteilung ist jeweils 100 %.

Gegenstand der Erfindung ist ebenso die Verwendung der beschriebenen Piperazinpolyphosphate als Flammschutzmittel, die Anwendung dieser Flammschutzmittel in Kunststoffen, Holz und Papier sowie die Anwendung in Beschichtungen für Stahl- und Holzbrandschutz.

Gegenstand der Erfindung ist zudem die Verwendung der beschriebenen Piperazinpolyphosphate
- als Binder z. B. für Gießereimassen und Formsande,
- als Vernetzer bzw. Beschleuniger beim Aushärten von Epoxyharzen, Polyurethanen, ungesättigten Polyesterharzen,
- als Polymerstabilisatoren, z. B. als Lichtschutzstabilisator und/oder Thermostabilisatoren für Baumwollgewebe, Polymerfasern, Kunststoffe. u. a.,
- als Pflanzenschutzmittel, z. B. als Pflanzenwachstumsregulator, als Herbizid, Pestizid, Fungizid, usw.
- als Therapeutikum oder Additiv in Therapeutika für Menschen und Tiere, z. B. als Enzymmodulator, zur Stimulierung von Gewebewachstum,
- als Sequestrierungsmittel z. B. zur Kontrolle von Ablagerungen in industriellen Wasserleitungssystemen, bei der Mineralölgewinnung und in Metallbehandlungsmitteln,
- als Mineralöl-Additiv z. B. als Antioxidans und zur Erhöhung der Octanzahl,
- als Korrosionsschutzmittel,
- in Wasch- und Reinigungsmittelanwendungen, z. B. als Entfärbungsmittel,
- in Elektronikanwendungen z. B. in Polyelektrolyten für Kondensatoren, Batterien und Akkumulatoren, sowie als Radikalfänger in photosensitiven Schichten.

Ferner Gegenstand der Erfindung ist die Anwendung der beanspruchten Piperazinpolyphosphate für pharmazeutische Zwecke.

Die nachfolgenden Beispiele dienen der Erläuterung der vorliegenden Erfindung, ohne den Gegenstand der Erfindung zu beschränken.

### Beispiele

### Beispiel 1

650 g Polyphosphorsäure mit einen P₂O₅ Gehalt von 81 % werden in einem Kneter vorgelegt und bei einer Temperatur von 115 °C wird portionsweise 383 g Piperazin zugegeben (Verhältnis Piperazin: Phosphorpentoxid 1,2:1). Nach beendeter Zugabe entsteht ein feines Pulver.

### Beispiel 2

175 g Polyphosphorsäure (81 %) werden in Toluol unter Rückfluss (110 °C) mit 86 g Piperazin versetzt. (Verhältnis Piperazin: Phosphorpentoxid 1:1) Es tritt sofort ein Niederschlag auf, der abfiltriert und im Vakuum von Lösungsmittelresten befreit wird. Ausbeute: 98,4 %

### Beispiel 3

668 g Polyphosphorsäure mit einem P₂O₅ Gehalt von 85 % werden bei 200 °C über eine Stunde gleichzeitig mit 344,4 g Piperazin (Verhältnis Piperazin: Phosphorpentoxid 1:1) in einen Kneter gegeben. Nach 10 Minuten Nachreaktionszeit wird das Piperazinpolyphosphat entnommen.

### Beispiel 4

86 g Piperazin werden mit 194 g Ammoniumpolyphosphat (Exolit^{®} AP 412, Clariant Produkte (Deutschland) GmbH) gemischt und in Wasser suspendiert. Dabei wird Ammoniak frei und das Wasser erwärmt sich. Nach 2 h wird 100 ml Methanol zugegeben und vom unlöslichen Niederschlag abfiltriert. Der Feststoff wird getrocknet.

### Beispiel 5

668 g Polyphosphorsäure mit einem P₂O₅-Gehalt von 86 % werden bei 230 °C über eine Stunde mit 348,5 g Piperazin (Verhältnis Piperazin-Phosphorpentoxid 1:1) in einem Kneter zur Reaktion gebracht. Nach 60 Minuten Temperzeit wird das Produkt entnommen. Mittels Ionenchromatographie wird die Kettenlänge 4 als Hauptprodukt identifiziert.

### Beispiel 6 (Vergleich)

Wie in DE-A-101 45 093 beschrieben, werden in einem Kneter 861 g (10 mol) Piperazin mit 710 g (5mol) Phosphorpentoxid gemischt und auf 100 °C aufgeheizt. (Molares Verhältnis Piperazin:Phosphorpentoxid 2:1).

Innerhalb von 15 Minuten werden dann 210 g (1,7 mol) Oxalsäuredihydrat zugegeben. Die Nachreaktionszeit beträgt eine Stunde, ehe das entstandene Piperazinpolyphosphat entnommen wird.

**Tabelle 1: Analysenwerte zu den Beispielen 1 bis 6**

| | Temperatur von 5 % GV [°C] | Kettenlänge (NMR) | Leitfähigkeit [µS/cm] | Säurezahl [mg KOH/g] | Wasserlöslichkeit [%] | Rest-feuchte |
|---|---|---|---|---|---|---|
| Bsp. 1 | 328 | 2,6 | 10240 | 328 | 2,1 | 0,16 |
| Bsp.2 | 331 | 2,4 | 11347 | 347 | 2,4 | 0,05 |
| Bsp.3 | 364 | 4,3 | 9100 | 265 | 4,3 | 0,09 |
| Bsp. 4 | 369 | 73 | 1853 | 3,0 | 0,3 | 0,72 |
| Bsp. 5 | 340 | 5,3 | 8800 | 251 | 3,7 | 0,08 |
| Bsp. 6 (Vergleich) | 151 | 1,5 | 51000 | n.b. | 10 | 0,17 |

Die erfindungsgemäßen Piperazinpolyphosphate der Beispiele 1 bis 5 weisen gegenüber dem Piperazinpolyphosphat nach dem Stand der Technik (Beispiel 6) vorteilhafterweise höhere Zersetzungstemperaturen, geringere Leitfähigkeiten und geringere Wasserlöslichkeiten auf.

## Patentansprüche

1. Piperazinpolyphosphat, **dadurch gekennzeichnet, dass** es eine mittlere Kettenlänge von 2,2 bis 10 Phosphateinheiten, eine Kettenlängenverteilung mit:
| | |
|---|---|
| Kettenlänge 1 | 0,5 bis 10 % |
| Kettenlänge 2 | 2 bis 30 % |
| Kettenlänge 3 | 4 bis 30 % |
| Kettenlänge 4 | 0 bis 90 % |
| Kettenlänge 5 | 1 bis 25 % |
| Kettenlänge 6 | 1 bis 25 % |
| Kettenlänge 7 | 1 bis 40 % |
| Kettenlänge 8 und darüber | 0,5 bis 40 %, |
eine 10 %ige Suspension davon in Wasser einen pH-Wert zwischen 1,5 und 7, die Säurezahl des Filtrats einer 10 %igen Suspension davon zwischen 200 und
400 mg KOH/g liegt und dass es eine Wasserlöslichkeit von maximal 7 % aufweist.

2. Piperazinpolyphosphat nach Anspruch 1, **dadurch gekennzeichnet, dass** es folgende Kettenlängenverteilung aufweist:
| | |
|---|---|
| Kettenlänge 1 | 0,5 bis 5 % |
| Kettenlänge 2 | 2 bis 20% |
| Kettenlänge 3 | 4 bis 20 % |
| Kettenlänge 4 | 30 bis 90 % |
| Kettenlänge 5 | 1 bis 15 % |
| Kettenlänge 6 | 1 bis 15 % |
| Kettenlänge 7 | 1 bis 30 % |
| Kettenlänge 8 und darüber | 0,5 bis 30 %. |

3. Piperazinpolyphosphat nach Anspruch 1 oder 2, **dadurch gekennzeichnet, dass** eine 10 %ige Suspension davon in Wasser einen pH-Wert zwischen 2 und 5,5 aufweist.

4. Piperazinpolyphosphat nach einem oder mehreren der Ansprüche 1 bis 3, **dadurch gekennzeichnet, dass** die Säurezahl des Filtrats einer 10%igen Suspension davon zwischen 200 und 400 mg KOH/g liegt.

5. Verfahren zur Herstellung von Piperazinpolyphosphat, **dadurch gekennzeichnet, dass** flüssiges und/oder gasförmiges Piperazin und Polyphosphorsäure miteinander umgesetzt werden.

6. Verfahren zur Herstellung von Piperazinpolyphosphat, **dadurch gekennzeichnet, dass** Polyphosphorsäure und Piperazin in einem Lösungsmittel miteinander umgesetzt werden.

7. Verfahren zur Herstellung von Piperazinpolyphosphat, **dadurch gekennzeichnet, dass** ein Salz einer Aminbase der Polyphosphorsäure in wässriger Lösung mit Piperazin umgesetzt wird.

8. Verwendung von Piperazinpolyphosphat nach mindestens einem oder mehreren der Ansprüche 1 bis 4 als Flammschutzmittel, insbesondere für Klarlacke und Intumeszenzbeschichtungen, Flammschutzmittel für Holz und andere cellulosehaltige Produkte, für oder in Kunststoffen, Holz, Papier und Textil sowie in Beschichtungen für Stahl- und Holzbrandschutz; als reaktives und/oder nicht reaktives Flammschutzmittel für Polymere, zur Herstellung von flammgeschützten Polymerformmassen, zur Herstellung von flamm-geschützten Polymerformkörpern, -Filmen, -Fäden und -Fasern und/oder zum flammhemmend Ausrüsten von Polyester und Cellulose-Rein- und Mischgeweben durch Imprägnierung.

9. Verwendung von Piperazinpolyphosphat nach mindestens einem oder mehreren der Ansprüche 1 bis 4
- als Zwischenprodukt für weitere Synthesen,
- als Binder,
- als Vernetzer bzw. Beschleuniger beim Aushärten von Epoxyharzen, Polyurethanen, ungesättigten Polyesterharzen,
- als Polymerstabilisatoren,
- als Pflanzenschutzmittel,
- als Therapeutikum oder Additiv in Therapeutika für Menschen und Tiere,
- als Sequestrierungsmittel,
- als Mineralöl-Additiv,
- als Korrosionsschutzmittel,
- in Wasch- und Reinigungsmittelanwendungen,
- in Elektronikanwendungen.

10. Flammgeschützte thermoplastische und/oder duroplastische Polymerformmasse, enthaltend
0,5 bis 45 Gew.-% Piperazinpolyphosphat nach mindestens einem oder mehreren der Ansprüche 1 bis 4, 0,5 bis 95 Gew.-% thermoplastisches Polymer oder Mischungen derselben, 0 bis 55 Gew.-% Additive und 0 bis 55 Gew.-% Füllstoff bzw. Verstärkungsmaterialien, wobei die Summe der Komponenten 100 Gew.-% beträgt.

11. Flammgeschützte thermoplastische und/oder duroplastische Polymer-Formkörper, -Filme, -Fäden und Fasern, enthaltend 0,5 bis 45 Gew.-% Piperazinpolyphosphat nach mindestens einem oder mehreren der Ansprüche 1 bis 4, 0,5 bis 95 Gew.-% thermoplastisches Polymer oder Mischungen derselben, 0 bis 55 Gew.-% Additive und 0 bis 55 Gew.-% Füllstoff bzw. Verstärkungsmaterialien, wobei die Summe der Komponenten 100 Gew.-% beträgt.
